# FASCICULE DE BREVET EUROPEEN

(11) **EP 2 890 674 B1**
(45) Date de publication et mention de la délivrance du brevet: **07.12.2016**
(21) Numéro de dépôt: 13762191.8
(22) Date de dépôt: 27.08.2013
(51) Int. Cl.: C07C 319/24, C07C 319/28, C07C 321/14

(54) **PRÉPARATION DE DISULFURES SYMÉTRIQUES ET DISSYMÉTRIQUES PAR DISTILLATION RÉACTIVE DE MÉLANGES DE DISULFURES**
HERSTELLUNG VON SYMMETRISCHEN UND ASYMMETRISCHEN DISULFIDEN DURCH REAKTIVE DESTILLATION VON DISULFIDGEMISCHEN
PRODUCTION OF SYMMETRIC AND ASYMMETRIC DISULPHIDES BY REACTIVE DISTILLATION OF DISULPHIDE MIXTURES

(30) Priorité: 30.08.2012 FR 1258110
(43) Date de publication de la demande: 08.07.2015
(73) Titulaire: Arkema France, 92700 Colombes (FR)
(72) Inventeur: FREMY, Georges, 64390 Sauveterre De Bearn (FR)
(86) Numéro de dépôt international: PCT/FR2013/051974
(87) Numéro de publication internationale: WO 2014/033399

(56) Documents cités:
- US-A- 2 510 893
- US-A- 2 521 870
- US-A- 2 557 312
- S.F. BIRCH, ET AL.: "Preparation and properties of dialkyl di- and poly-sulfides; some disproportionation reactions", JOURNAL OF THE INSTITUTE OF PETROLEUM, vol. 39, no. 352, avril 1953 (1953-04), pages 206-219, XP008162466, London, GB ISSN: 0020-3068
- D.T. MCALLAN, ET AL.: "The preparation and properties of sulfur compounds related to petroleum. I. The dialkyl sulfides and disulfides", JOURNAL OF THE AMERICAN CHEMICAL SOCIETY, vol. 73, no. 8, 6 août 1951 (1951-08-06), pages 3627-3632, XP002055386, American Chemical Society, Washington, DC, US ISSN: 0002-7863, DOI: 10.1021/ja01152a021

## Description

La présente invention concerne un procédé de production de disulfures de dialkyle, symétriques ou dissymétriques par distillation réactive de mélanges de disulfures organiques (tels que les DSO, ou « DiSulfide Oils » en langue anglaise). Les DSO sont des mélanges de disulfures provenant par exemple des champs d'extraction de gaz ou de pétrole.

Plus précisément, la présente invention concerne le domaine des disulfures de dialkyle, en particulier des disulfures symétriques, et plus particulièrement celui du disulfure de diméthyle et du disulfure de diéthyle.

Dans la suite du présent exposé, on entend par disulfures et polysulfures de dialkyle les composés de formule générale (1) :

R-Sₙ-R' (1)

dans laquelle :
- R et R', identiques ou différents, représentent, chacun indépendamment l'un de l'autre, un radical hydrocarboné, linéaire ou ramifié, contenant de 1 à 12 atomes de carbone, de préférence de 1 à 8 atomes de carbone, et éventuellement une ou plusieurs insaturations sous forme de double(s) et/ou triple(s) liaison(s) ;
- n représente un entier compris entre 2 et 8, de préférence entre 2 et 6, de préférence encore entre 2 et 4, bornes incluses.

Les disulfures de dialkyle sont ainsi représentés par la formule générale (1), dans laquelle n est égal à 2. Les polysulfures de dialkyle sont représentés par la formule générale (1), dans laquelle n est strictement supérieur à 2.

Les disulfures et polysulfures de dialkyle de formule (1) sont dits symétriques lorsque les radicaux R et R' sont identiques, et sont dits dissymétriques lorsque R et R' sont différents. Par « identiques », on entend de même nombre d'atomes de carbone, et dans la même configuration stéréochimique. Dans le cadre de la présente invention, on préfère les disulfures de dialkyle de formule (1) (où n = 2) ci-dessus pour lesquels R et R', identiques ou différents, représentent une chaîne hydrocarbonée, linéaire ou ramifiée, contenant de 1 à 12 atomes de carbone, de préférence de 1 à 8 atomes de carbone, et qui est saturée (radical alkyle).

Parmi les radicaux alkyle qui peuvent former les radicaux R et R', on préfère les radicaux comportant de 1 à 8 atomes de carbone, de préférence de 1 à 6 atomes de carbone, par exemple les radicaux méthyle, éthyle, n-propyle, *iso-*propyle, n-butyle, *iso*-butyle, *tert*-butyle, n-pentyle, *neo*-pentyle et n-hexyle.

Dans le contexte de la présente invention, on préfère les disulfures de dialkyle choisis parmi le disulfure de diméthyle (ou diméthyldisulfure ou DMDS), le disulfure de diéthyle (ou diéthyldisulfure ou DEDS), le disulfure de dipropyle (ou dipropyldisulfure ou DPDS), le disulfure de dibutyle (ou dibutyldisulfure ou DBDS), le disulfure de dipentyle (ou disulfure de diamyle), le disulfure de dihexyle, les homologues trisulfures (n = 3), les homologues tétrasulfures (n = 4), les homologues pentasulfures (n = 5), les homologues hexasulfures (n = 6), de ces disulfures, ainsi que leurs mélanges, étant entendu que les radicaux propyle et butyle peuvent se présenter sous forme linéaire ou ramifiée, par exemple, n-propyle, iso-propyle, n-butyle, iso-butyle, sec-butyle et tert-butyle, n-pentyle, sec-pentyle, neo-pentyle, et autres.

Parmi les disulfures de dialkyle, le disulfure de diméthyle (diméthyldisulfure, DMDS) est un composé aujourd'hui très largement utilisé dans de très nombreux domaines de l'industrie, par exemple industries chimiques, industries pétrochimiques, en agrochimie, dans l'industrie du bâtiment, et autres. Le DMDS est notamment utilisé comme agent de sulfuration de catalyseurs d'hydrotraitement de charges pétrolières ou en tant qu'additif de charge pour le vapocraquage.

Par rapport à d'autres produits utilisés dans ces applications tels que les *tertio*-alkylpolysulfures commerciaux, le DMDS présente de nombreux avantages, notamment une teneur en soufre élevée (68 %) et des produits de dégradation non cokants (CH₄, H₂S). De plus, dans ces applications, le DMDS conduit à des performances généralement supérieures aux autres produits tels que les *tertio-*alkylpolysulfures.

Parmi les méthodes de synthèse du DMDS, une méthode particulièrement efficace et économique est l'oxydation du méthylmercaptan par le soufre selon la réaction :

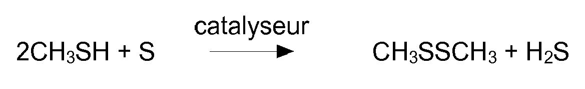

Cette réaction est catalysée par des agents basiques organiques ou inorganiques, homogènes ou hétérogènes. Cette voie est particulièrement économique quand on considère que de l'H₂S est coproduit et que ce dernier peut être utilisé pour réaliser la synthèse du méthylmercaptan par réaction avec le méthanol selon la réaction suivante :

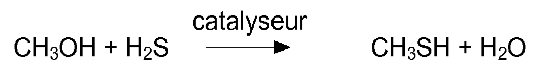

Il existe d'autres voies de synthèse du DMDS décrites dans l'art antérieur telles que l'oxydation du méthylmercaptan par l'eau oxygénée ou l'oxygène de l'air.

Il a maintenant été trouvé une autre voie pour la production de DMDS, et plus généralement de disulfures de dialkyle symétriques ou dissymétriques, cette autre voie de synthèse présentant un avantage économique au moins équivalent à celui apporté par le procédé d'oxydation du méthylmercaptan par le soufre, et présentant en outre le grand avantage de permettre une valorisation tout à fait intéressante des DSO.

Les DSO (« DiSulfide Oils » en langue anglaise) sont des mélanges de disulfures organiques produits lors du traitement des mercaptans contenus dans des coupes pétrolières ou des gaz liquéfiés par des procédés de type « Merox » (voir par exemple Catal. Review- Sci. Eng., 35(4), (1993), 571-609).

Ces DSO sont généralement et le plus souvent éliminés, détruits, stockés, selon différentes techniques, sur le site même de production, notamment sur les sites gaziers par exemple dans les unités de récupération de soufre. Une des techniques de destruction des DSO couramment utilisée est celle basée sur la réaction de Claus, qui permet l'élimination conjointe du sulfure d'hydrogène (H₂S) contenu également dans le gaz naturel brut.

La réaction de Claus peut s'écrire selon la réaction (A) suivante :
(A)

   2 H₂S + SO₂ → 3 S + 2 H₂O,

   le dioxyde de soufre (SO₂) étant préparé par oxydation d'une partie de l'H₂S, selon la réaction (B) suivante :
(B)

   H₂S + 3/2 O₂ → SO₂ + H₂O

   et également par traitement oxydant des DSO, selon la réaction (C) suivante, où les DSO sont illustrés par le DMDS :
(C)

   CH₃SSCH₃ + 11/2 O₂ → 2 CO₂ + 2 SO₂ + 3 H₂O.

Le problème est que les réactions (A) et (B) sont réalisées avec un défaut d'oxygène alors que la réaction (C) nécessite un excès d'oxygène par rapport à la stoechiométrie pour être complète, car la présence d'imbrûlés carbonés dans la réaction de Claus cause de sérieux problèmes de qualité du soufre obtenu par cette réaction. La quantité de DSO qu'il est donc possible de traiter conjointement à l'incinération d'H₂S est très limitée et souvent inférieure à la quantité produite quotidiennement sur le site gazier concerné.

La demande de brevet FR 2 875 236 A décrit très bien les problèmes rencontrés avec cette technique d'élimination et ses variantes. Cette demande propose également une autre méthode de destruction basée sur l'hydrogénolyse complète de ces disulfures en hydrocarbures et H₂S, puis incinération de ce dernier dans une unité Claus.

Dans le but de valoriser plutôt que de détruire les DSO, la demande internationale WO 2005/111175 propose d'utiliser ces produits comme inhibiteurs de formation de coke dans les fours de craquage d'hydrocarbures. Il est en effet connu par l'homme du métier que l'ajout de composés soufrés (comme par exemple le DMDS) dans les charges d'hydrocarbures permet de réduire fortement la formation de coke dans les tubes des vapocraqueurs, par exemple.

Cependant, ainsi que le mentionne la demande internationale WO 2001/96499, les DSO sont formés par oxydation des sels de sodium des mercaptans à éliminer et contiennent, de ce fait, des traces de sodium en quantité significative. Cela a pour conséquence l'impossibilité d'une utilisation directe, sans traitement préalable, dans des fours de craquage car la présence de sodium modifie la métallurgie des tubes de craquage. Par ailleurs, l'utilisation des DSO dans une autre application telle que la sulfuration des catalyseurs d'hydrotraitement de coupes pétrolières est également compromise car le sodium est connu pour être un poison de ces catalyseurs.

La valorisation directe de ces DSO comme intermédiaires de synthèse est également contrariée par le fait, cette fois, que ces DSO sont des mélanges de disulfures et non des molécules chimiquement pures telles que le disulfure de diméthyle ou le disulfure de diéthyle, pris isolément. Par exemple, un DSO issu du traitement d'un gaz de pétrole liquéfié (GPL) contient du disulfure de diméthyle, du disulfure de diéthyle et une quantité importante de disulfure d'éthylméthyle.

D'autres procédés concernent plus spécifiquement les disulfures symétriques et dissymétriques. Ainsi, le brevet US 2 521 870 divulgue un procédé permettant de préparer des disulfures dissymétriques à partir d'un mélange d'au moins deux disulfures symétriques différents (« disproportionation »), par chauffage, en présence de sels de sodium. Il est également connu du brevet US 2 557 312 de préparer des disulfures symétriques à partir de disulfures dissymétriques (« reproportionation ») à partir d'un mélange d'au moins deux disulfures dissymétriques, par chauffage, en présence d'une alcanolamine et d'un sulfure de métal alcalin.

Par ailleurs, une solution évidente pour l'homme de l'art désireux de produire des disulfures de dialkyle purs, symétriques et non mélangés à partir des DSO, consisterait à séparer les différents disulfures de dialkyle par une distillation en fonction des points d'ébullition. Cependant, cette technique ne permettrait pas de valoriser les disulfures de dialkyle dissymétriques en disulfures de dialkyle symétriques, tels que le disulfure d'éthylméthyle présent dans les DSO issus de GPL, ce disulfure dissymétrique étant même parfois le composé majoritaire.

Il en va de même pour les DSO plus complexes (mélanges complexes de disulfures de dialkyle issus de condensats par exemple) dans lesquels sont présents des disulfures ayant des groupes alkyles de rangs inférieurs, tels que des groupes alkyle en C₁, C₂, C₃, C₄, voire de rangs supérieurs. Dans ces DSO, les disulfures symétriques peuvent être en quantités relativement faibles par rapport aux disulfures dissymétriques au vu des différentes combinaisons possibles (C₁/C₂, C₁/C₃, C₁/C₄, C₂/C₃,...) et la possibilité d'isomères structuraux à partir du rang C₃ (isopropyle/n-propyle).

Ainsi, outre le premier objectif de proposer une variante particulièrement économique du procédé de préparation spécifique de DMDS, un autre objectif de la présente invention est de proposer, de manière plus générale un procédé de valorisation des DSO. Un autre objectif encore de la présente invention consiste à récupérer et produire de façon avantageuse des disulfures de dialkyle purs, non mélangés, symétriques ou dissymétriques, avec un rendement amélioré, voire maximal. D'autres objectifs encore apparaîtront dans l'exposé de l'invention qui suit.

Il a maintenant été découvert qu'il est possible d'atteindre ces objectifs, au moins en partie, voire en totalité, grâce au procédé de la présente invention.

Ainsi, et selon un premier aspect, l'invention concerne un procédé de préparation de disulfures de dialkyle symétriques ou dissymétriques en engageant au moins un DSO dans une réaction catalytique basique et éventuellement photochimique, avec extraction conjointe, de préférence en continu du ou des disulfure(s) symétrique(s) et/ou dissymétrique(s) que l'on souhaite obtenir.

Plus précisément, le procédé de l'invention concerne un procédé de préparation de disulfures de dialkyle symétriques ou dissymétriques, ledit procédé comprenant au moins les étapes suivantes :
a) introduction dans un réacteur d'au moins un mélange d'au moins deux disulfures organiques de formule générale (1) : R-Sₙ-R', dans laquelle :
   - R et R', identiques ou différents, représentent, chacun indépendamment l'un de l'autre, un radical hydrocarboné, linéaire ou ramifié, contenant de 1 à 12 atomes de carbone, de préférence de 1 à 8 atomes de carbone, et éventuellement une ou plusieurs insaturations sous forme de double(s) et/ou triple(s) liaison(s) ; et
   - n représente 2,
b) conduite d'une réaction catalytique basique et éventuellement photochimique, au cours de laquelle les composés de formule (1) sont transformés par reproportionation et par disproportionation en un mélange de disulfures de dialkyle symétriques et/ou dissymétriques,
c) extraction, de préférence en continu, du ou des disulfure(s) de dialkyle symétrique(s) et/ou dissymétrique(s) souhaité(s),
ledit catalyseur étant un catalyseur sur support solide imprégné d'au moins un oxyde d'alcalin, d'alcalino-terreux ou d'un oxyde métallique, ledit support solide étant choisi parmi alumine, silice, zéolithes, charbons, charbons actifs, argiles, zircones, dioxyde de titane, résines polymères échangeuses d'ions, de préférence échangeuses d'anions, faiblement ou fortement basiques.

Il doit être compris que ledit catalyseur basique permet de catalyser à la fois les réactions de disproportionation et les réactions de reproportionation.

Comme catalyseur basique solide approprié pour une catalyse basique hétérogène, il est possible d'utiliser les catalyseurs dont le support solide est imprégné d'au moins un oxyde d'alcalin, d'alcalino-terreux ou d'un oxyde métallique, tels que par exemple Na₂O, K₂O, MgO, CaO, et autres, et leurs mélanges. Parmi les supports solides, on peut citer par exemple Amberlyst^{®} A21 de Dow Chemicals.

Un catalyseur tout à fait adapté au procédé de l'invention selon le mode catalyse basique hétérogène, est l'oxyde de sodium (Na₂O) supporté sur alumine, et commercialisé par la société Axens sous le nom CM2-5.

Selon une variante, la réaction catalytique du procédé de la présente invention, peut être une réaction photochimique. Il doit être également compris que cette réaction photochimique permet de catalyser à la fois les réactions de disproportionation et les réactions de reproportionation.

On utilise généralement dans ce cas une source de rayonnement compris dans la gamme des ultraviolets, entre environ 260 nm et environ 350 nm. Une lampe tout particulièrement adaptée pour la réaction de catalyse photochimique du procédé de la présente invention est une lampe ultraviolette de longueur d'onde 265 nm, permettant une photolyse directe et rapide des disulfures, même sans l'aide de photo-initiateurs qui génèrent des réactions généralement plus lentes.

La réaction catalytique du procédé de l'invention peut combiner une catalyse basique avec catalyseur supporté et une catalyse photochimique. La réaction de catalyse basique et éventuellement photochimique (étape b)) peut être conduite à toute température appropriée et adaptée selon les connaissances de l'homme du métier. Par exemple les températures adaptées pour la conduite de la réaction selon le procédé de la présente invention peuvent être comprises entre environ -10°C à + 300°C, de préférence entre +20°C et +200°C.

La pression réactionnelle peut également varier dans de grandes proportions, selon notamment la température de réaction, les réactants et les produits souhaités, et peut varier de 1 mbar (vide poussé) à plusieurs dizaines de bars, par exemple de 10 mbars (vide léger) à environ 3 bars. Selon un mode de réalisation préféré, le procédé de la présente invention est conduit à pression atmosphérique, ou sous vide léger ou sous vide poussé.

Le procédé selon l'invention permet ainsi d'obtenir, à partir de mélanges de disulfures symétriques et/ou dissymétriques, tels que par exemple ceux présents dans les DSO, de manière simple, rapide et avec de bons rendements, des disulfures symétriques et/ou dissymétriques de haute pureté.

Dans le milieu réactionnel, et après conduite de la réaction catalytique, les disulfures symétriques et/ou dissymétriques formés sont les produits de reproportionation et de disproportionation des disulfures de départ, et par conséquent ledit milieu réactionnel contient, outre les produits de départ, d'autres disulfures symétriques et/ou dissymétriques.

Le schéma suivant illustre une des réactions possibles, dans laquelle un disulfure dissymétrique conduit à deux disulfures symétriques (reproportionation) et vice-versa (disproportionation), selon la réaction (D) suivante :

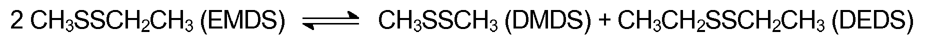

Cette réaction est une réaction équilibrée, et l'équilibre peut être déplacé par extraction, de préférence en continu, de l'un ou des produits que l'on souhaite obtenir.

En reprenant le schéma ci-dessus, un autre exemple consiste utiliser un mélange de EMDS (disulfure dissymétrique) et de disulfure de dibutyle (DBDS, disulfure symétrique). Selon le procédé de l'invention, le schéma réactionnel peut ainsi s'écrire : 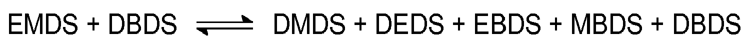 schéma dans lequel EBDS est le disulfure d'éthyle et de butyle, MBDS est le disulfure de méthyle et de butyle et DBDS est le disulfure de dibutyle. Cet exemple montre qu'à partir d'un mélange de deux disulfures, il est possible d'obtenir quatre autres disulfures.

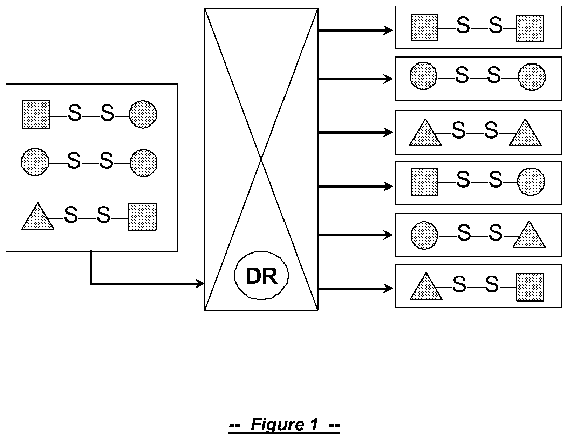

La Figure 1 donne une représentation schématique des disulfures qui peuvent être formés, dans le réacteur DR (qui illustre une colonne de distillation réactive), à partir d'un mélange de trois disulfures (deux disulfures symétriques et un disulfure dissymétrique), dans lesquels les symboles ■, ●, et ▲ représentent chacun un radical alkyle choisi parmi R et R' définis précédemment. Les réactions de disproportionation et de reproportionation catalytiques et éventuellement photochimiques décrites ci-dessus sont toutes des réactions équilibrées, et le ou les disulfures symétriques ou dissymétriques d'intérêts peuvent être aisément extraits du milieu réactionnel, ceci ayant pour effet bien connu de déplacer l'équilibre réactionnel.

L'extraction (étape c)) du ou des disulfures symétriques ou dissymétriques d'intérêt peut être réalisée selon toute méthode, ou toutes combinaisons de méthodes, connues de l'homme du métier, parmi lesquelles on peut citer par exemple le soutirage, la distillation, ces deux méthodes étant préférées, séparément ou en combinaison. Ainsi, et selon un mode de réalisation tout particulièrement préféré, le procédé de la présente invention peut être réalisé dans une colonne de type colonne de distillation, comprenant le catalyseur basique et/ou pourvue d'un équipement permettant la photocatalyse.

Ainsi, le réacteur de disproportionation et de reproportionation permet en même temps l'extraction du ou des disulfures symétriques ou dissymétriques d'intérêt par simple distillation. Ainsi, les réactions étant équilibrées comme indiqué précédemment, le bouilleur de la distillation ou le pied de colonne s'enrichit progressivement en disulfure le moins volatil (par exemple DEDS, dans la réaction (D) ci-dessus) alors que la tête de distillation s'enrichit en disulfure le plus volatil (DMDS, dans la réaction (D) ci-dessus).

La technique de distillation est une technique bien connue de l'homme du métier et peut être effectuée dans une colonne à distiller, en batch ou en continu. On préfère effectuer l'extraction du ou des produits souhaités dans une colonne de distillation, par exemple dans les conditions décrites dans le brevet US 2 557 312, selon la nature des disulfures ou des mélanges de disulfures en présence, ainsi que selon la nature des produits que l'on souhaite obtenir. L'opération de distillation peut ainsi être opérée sous pression atmosphérique, de préférence sous vide léger ou poussé, de manière à éviter des températures de distillation supérieures à environ 200°C.

Typiquement, le procédé de la présente invention comprend une étape réactionnelle catalytique de disproportionation et/ou de reproportionation, conjointement avec une étape d'extraction, préférentiellement par distillation, du ou des produits réactionnels souhaités, permettant de déplacer l'équilibre de ladite réaction catalytique. Ce procédé peut ainsi être défini comme une « distillation réactive » de disulfure(s) de dialkyle.

Selon un mode de réalisation préféré du procédé de l'invention, le mélange de disulfures, par exemple un mélange de DSO, après traitement par catalyse basique et éventuellement catalyse photochimique, est distillé afin de déplacer l'équilibre représentée dans les réactions ci-dessus vers la droite, au fur et à mesure du soutirage du ou des disulfures de dialkyle symétriques ou dissymétriques les plus léger, le ou les disulfures de dialkyle symétriques les plus lourd restant dans le bouilleur ou en pied de colonne.

Dans le mode de distillation réactive exposé précédemment, le ou les produits les plus légers, extraits sous forme gazeuse, sont liquéfiés, selon les techniques classiques connues de l'homme du métier, par exemple par refroidissement.

Les composés les plus lourds restant dans le bouilleur peuvent être également séparés par augmentation de la température et/ou diminution de la pression, afin de poursuivre la séparation desdits composés. Le composé le plus lourd restant dans le bouilleur est ainsi obtenu sous forme pure contrairement à se qui serait obtenu en mettant en oeuvre les procédés décrits dans l'art antérieur.

Le procédé de la présente invention peut être effectué en batch ou en continu. On préfère conduire le procédé de l'invention en batch lorsqu'il s'agit de traiter un lot de mélanges de disulfures que l'on peut charger en une fois dans le réacteur, et on préfère conduire le procédé de l'invention en continu lorsque le réacteur est alimenté en continu par un flux de mélange de disulfures provenant d'un site d'extraction générant de tels mélanges, par exemple les mélanges de disulfures (DSO) produits lors du traitement des mercaptans contenus dans des coupes pétrolières ou des gaz liquéfiés par les procédés de type « Merox ».

Il est possible de réaliser tout d'abord la réaction catalytique (basique et éventuellement photochimique, comme indiqué précédemment) puis l'extraction du ou des disulfures symétriques ou dissymétriques d'intérêt, ou bien de combiner la réaction catalytique (basique et éventuellement photochimique, comme indiqué précédemment) et l'extraction du ou des disulfures symétriques ou dissymétriques d'intérêt, cette variante étant tout particulièrement préférée. Dans ce cas le procédé de l'invention est opéré dans une colonne dite de « distillation réactive », combinant les réactions de reproportionation et/ou de disproportionation ainsi que l'extraction par distillation du ou des disulfures symétriques ou dissymétriques d'intérêt.

Dans un mode de réalisation tout particulièrement adapté du procédé de la présente invention, la distillation réactive est opérée en continu, ce qui offre l'avantage d'obtenir des disulfures de dialkyle, par exemple des disulfures de dialkyle symétriques, par exemple le DMDS, purs (puisque déjà distillés), et directement utilisables.

Un autre avantage du procédé de la présente invention réside dans le fait que les disulfures de dialkyle ainsi obtenus directement par distillation réactive de DSO sont débarrassés des impuretés, minérales et organiques présentes dans les DSO.

Grâce au procédé de la présente invention, les rendements en disulfures de dialkyle purs obtenus sont supérieurs à ceux que l'on aurait obtenus avec une distillation simple des DSO, le dit procédé de l'invention permettant de transformer la grande majorité, voire la totalité des disulfures dissymétriques en disulfures symétriques.

Selon une variante avantageuse du procédé selon la présente invention, la réaction catalytique peut être conduite directement dans la colonne de distillation. Un mode de réalisation de cette variante préférée comprend un lit de catalyseur basique intégré dans la colonne de distillation.

Le procédé de la présente invention est tout particulièrement adapté à la préparation de disulfures de dialkyle symétriques « purs », à partir de DSO, mais peut également s'appliquer à la préparation de disulfures de dialkyle dissymétriques « purs » à partir de DSO, en opérant une distillation spécifique, par déplacement de la stoechiométrie (soutirage/extraction du ou des disulfures symétriques ou dissymétriques d'intérêt), avec un appareillage adapté.

Ainsi, la présente invention fournit un procédé tout particulièrement adapté à la valorisation des DSO par distillation réactive pour conduire, en une étape de distillation réactive, à un ou plusieurs disulfures de dialkyle, symétriques et/ou dissymétriques, purs et directement utilisables, sans nécessiter d'opération de purification supplémentaire.

Selon un aspect tout particulièrement préféré de la présente invention, le procédé décrit précédemment permet, à titre d'exemple non limitatif, la préparation de disulfure de diméthyle (DMDS), plus précisément le procédé de la présente invention concerne la synthèse de DMDS par distillation réactive, à partir d'un DSO, la distillation étant conduite à pression atmosphérique, la température du bouilleur étant d'environ 110°C-150°C, par exemple 130°C. Le DMDS est alors soutiré en tête de colonne à son point d'ébullition qui est 109°C à pression atmosphérique.

Selon un autre aspect le procédé de la présente invention permet la préparation de disulfure de diéthyle, plus précisément le procédé de la présente invention concerne la synthèse de DEDS par distillation réactive, à partir d'un DSO, la distillation étant conduite à pression atmosphérique, la température du bouilleur étant d'environ 160-180°C, par exemple 170°C. Le DEDS est alors soutiré à son tour en tête de colonne à son point d'ébullition qui est 154°C à pression atmosphérique.

On comprendra mieux l'invention avec les exemples suivants :

### EXEMPLE 1 : (comparatif)

Soit 152 g de mélange de disulfures contenant 89,4 g de DMDS (disulfure de diméthyle), 55,1 g de EMDS (disulfure d'éthylméthyle) et 7,5 g de DEDS (disulfure de diéthyle).

La distillation de ce mélange, en l'absence de catalyse basique ou photochimique, conduit à récupérer 88,5 g de DMDS soit 99% de la quantité initialement présente.

### EXEMPLE 2

Soit 152 g du mélange de l'exemple 1 auquel on ajoute 2% d'une solution aqueuse d'hydroxyde de sodium à 50 % en poids. L'ensemble est engagé dans une distillation réactive selon la présente invention.

On soutire en continu le DMDS en tête de colonne que l'on liquéfie par refroidissement selon les techniques classiques connues de l'homme du métier. La quantité de DMDS récupérée après distillation est de 98,3 g et représente 110% de la quantité initialement présente.

### EXEMPLE 3

Soit 321,5 g d'un autre DSO, mélange relativement pauvre en DMDS initialement (DMDS 5% en poids, EMDS 34,5% et DEDS 60,5%). Exprimé en poids, ce mélange contient donc 16,1 g de DMDS, 110,9 g de EMDS et 194,5 g de DEDS. On effectue la distillation réactive selon la présente invention en présence de 50 g d'une alumine dopée avec 3% de Na₂O (catalyseur CM 2-5 de la société Axens).

La quantité de DMDS récupérée après distillation est de 63 g et représente 390% de la quantité initialement présente.

L'analyse du contenu du bouilleur montre qu'il contient 255,2 g de DEDS et 3,3 g de EMDS. Le taux de conversion de l'EMDS (dissymétrique) est donc de 97%.

## Revendications

1. Procédé de préparation de disulfures de dialkyle symétriques ou dissymétriques, ledit procédé comprenant au moins les étapes suivantes :
a) introduction dans un réacteur d'au moins un mélange d'au moins deux disulfures organiques de formule générale (1) : R-Sₙ-R', dans laquelle :
• R et R', identiques ou différents, représentent, chacun indépendamment l'un de l'autre, un radical hydrocarboné, linéaire ou ramifié, contenant de 1 à 12 atomes de carbone, de préférence de 1 à 8 atomes de carbone, et éventuellement une ou plusieurs insaturations sous forme de double(s) et/ou triple(s) liaison(s) ; et
• n représente 2,
b) conduite d'une réaction catalytique basique et éventuellement photochimique, au cours de laquelle les composés de formule (1) sont transformés par reproportionation et par disproportionation en un mélange de disulfures de dialkyle symétriques et/ou dissymétriques,
c) extraction, de préférence en continu, du ou des disulfure(s) de dialkyle symétrique(s) et/ou dissymétrique(s) souhaité(s), ledit catalyseur basique étant un catalyseur sur support solide imprégné d'au moins un oxyde d'alcalin, d'alcalino-terreux ou d'un oxyde métallique, ledit support solide étant choisi parmi alumine, silice, zéolithes, charbons, charbons actifs, argiles, zircones, dioxyde de titane, résines polymères échangeuses d'ions, de préférence échangeuses d'anions, faiblement ou fortement basiques.

2. Procédé selon la revendication 1, dans lequel le catalyseur est l'oxyde de sodium (Na₂O) supporté sur alumine.

3. Procédé selon la revendication 1 ou la revendication 2, combinant une catalyse basique et une catalyse photochimique.

4. Procédé selon l'une quelconque des revendications précédentes, dans lequel le photocatalyseur est un photocatalyseur de rayonnement compris entre environ 260 nm et environ 350 nm.

5. Procédé selon l'une quelconque des revendications précédentes, dans lequel l'étape b) est conduite à une température comprise entre environ -10°C à + 300°C, de préférence entre +20°C et +200°C.

6. Procédé selon l'une quelconque des revendications précédentes, dans lequel l'étape c) d'extraction comprend le soutirage ou la distillation ou une combinaison de ces deux techniques.

7. Procédé selon l'une quelconque des revendications précédentes, **caractérisé en ce qu'**il est réalisé dans une colonne de type colonne de distillation, comprenant le catalyseur basique et/ou pourvue d'un équipement permettant la photocatalyse.

8. Procédé selon l'une quelconque des revendications précédentes, **caractérisé en ce qu'**il est réalisé en batch ou en continu, de préférence en continu.

9. Procédé selon l'une quelconque des revendications précédentes, pour la préparation de disulfure de diméthyle.

10. Procédé selon l'une quelconque des revendications 1 à 8, pour la préparation de disulfure de diéthyle.

## Patentansprüche

1. Verfahren zur Herstellung von symmetrischen oder unsymmetrischen Dialkyldisulfiden, wobei das Verfahren mindestens die folgenden Schritte umfasst:
a) Eintragen mindestens einer Mischung von mindestens zwei organischen Disulfiden der allgemeinen Formel (1): R-Sₙ-R' in ein Reaktionsgefäß, wobei:
• R und R' gleich oder verschieden sind und jeweils unabhängig voneinander für einen linearen oder verzweigten Kohlenwasserstoffrest mit 1 bis 12 Kohlenstoffatomen, vorzugsweise 1 bis 8 Kohlenstoffatomen, und gegebenenfalls einer oder mehreren Ungesättigtheiten in Form von Doppel- und/oder Dreifachbindungen, stehen und
• n für 2 steht,
b) Durchführen einer basischen und gegebenenfalls photochemischen katalytischen Reaktion, in deren Verlauf die Verbindungen der Formel (1) durch Reproportionierung und durch Disproportionierung in einem Gemisch von symmetrischen und/oder unsymmetrischen Dialkyldisulfiden umgewandelt werden,
c) Extrahieren, vorzugsweise kontinuierliches Extrahieren, des bzw. der gewünschten symmetrischen und/oder unsymmetrischen Dialkyldisulfide, wobei es sich bei dem basischen Katalysator um einen mit mindestens einem Alkalimetalloxid, Erdalkalimetalloxid oder Metalloxid imprägnierten festen Träger handelt, wobei der feste Träger aus Aluminiumoxid, Siliciumdioxid, Zeolithen, Kohlen, Aktivkohlen, Tonen, Zirconiumdioxiden, Titandioxid und Ionenaustauscherpolymerharzen, vorzugsweise Anionenaustauscherpolymerharzen, die schwach oder stark basisch sind, ausgewählt ist.

2. Verfahren nach Anspruch 1, wobei es sich bei dem Katalysator um auf Aluminiumoxid geträgertes Natriumoxid (Na₂O) handelt.

3. Verfahren nach Anspruch 1 oder Anspruch 2 unter Kombination einer basischen Katalyse und einer photochemischem Katalyse.

4. Verfahren nach einem der vorhergehenden Ansprüche, bei dem es sich bei dem Photokatalysator um einen Photokatalysator für Strahlung zwischen ungefähr 260 nm und ungefähr 350 nm handelt.

5. Verfahren nach einem der vorhergehenden Ansprüche, bei dem Schritt b) bei einer Temperatur zwischen ungefähr -10°C bis +300°C, vorzugsweise zwischen +20°C und +200°C, durchgeführt wird.

6. Verfahren nach einem der vorhergehenden Ansprüche, bei dem der Extraktionsschritt c) Abziehen oder Destillieren oder eine Kombination dieser beiden Techniken umfasst.

7. Verfahren nach einem der vorhergehenden Ansprüche, **dadurch gekennzeichnet, dass** es in einer Kolonne von Destillationskolonnentyp, die den basischen Katalysator umfasst und/oder mit einer Einrichtung, die die Photokatalyse ermöglicht, ausgestattet ist, durchgeführt wird.

8. Verfahren nach einem der vorhergehenden Ansprüche, **dadurch gekennzeichnet, dass** es diskontinuierlich oder kontinuierlich, vorzugsweise kontinuierlich, durchgeführt wird.

9. Verfahren nach einem der vorhergehenden Ansprüche zur Herstellung von Dimethyldisulfid.

10. Verfahren nach einem der Ansprüche 1 bis 8 zur Herstellung von Diethyldisulfid.

## Claims

1. Process for the preparation of symmetrical or asymmetrical dialkyl disulphides, the said process comprising at least the following stages:
a) introduction, into a reactor, of at least one mixture of at least two organic disulphides of general formula (1) : R-Sₙ-R', in which:
• R and R', which are identical or different, represent, each independently of one another, a linear or branched hydrocarbon radical comprising from 1 to 12 carbon atoms, preferably from 1 to 8 carbon atoms, and optionally one or more unsaturations in the form of double and/or triple bond(s); and
• n represents 2,
b) carrying out a basic and optionally photochemical catalytic reaction, during which the compounds of formula (1) are converted, by reproportionation and by disproportionation, into a mixture of symmetrical and/or asymmetrical dialkyl disulphides,
c) extraction, preferably continuously, of the symmetrical and/or asymmetrical dialkyl disulphide(s) desired, the said basic catalyst being a catalyst on a solid support impregnated with at least one alkali metal or alkaline earth metal oxide or with a metal oxide, the said solid support being chosen from alumina, silica, zeolites, charcoals, active charcoals, clays, zirconias, titanium dioxide or ion-exchange polymer resins, preferably anion-exchange polymer resins, which are weakly or strongly basic.

2. Process according to Claim 1, in which the catalyst is sodium oxide (Na₂O) suuported on alumina.

3. Process according to Claim 1 or Claim 2, combining a basic catalysis and a photochemical catalysis.

4. Process according to any one of the preceding claims, in which the photocatalyst is a photocatalyst for radiation of between approximately 260 nm and approximately 350 nm.

5. Process according to any one of the preceding claims, in which stage b) is carried out at a temperature of between approximately -10°C and +300°C, preferably between +20°C and +200°C.

6. Process according to any one of the preceding claims, in which the extraction stage c) comprises withdrawal or distillation or a combination of these two techniques.

7. Process according to any one of the preceding claims, **characterized in that** it is carried out in a column of distillation column type, comprising the basic catalyst and/or provided with equipment which makes possible photocatalysis.

8. Process according to any one of the preceding claims, **characterized in that** it is carried out batchwise or continuously, preferably continuously.

9. Process according to any one of the preceding claims, for the preparation of dimethyl disulphide.

10. Process according to any one of Claims 1 to 8, for the preparation of diethyl disulphide.
